# EUROPEAN PATENT APPLICATION

(11) **EP 3 124 520 A1**
(43) Date of publication of application: **01.02.2017**
(21) Application number: 15768224.6
(22) Date of filing: 26.03.2015
(51) Int. Cl.: C08G 63/199, C07C 31/20, C08G 18/42

(54) **POLYESTER POLYOL HAVING ALICYCLIC SKELETON**

(30) Priority: 28.03.2014 JP 2014070036
(71) Applicant: Kuraray Co., Ltd., Okayama 710-0801 (JP)
(72) Inventor: TSURUTA Takuo, Tainai-shi Niigata 959-2691 (JP); OKANO Shigeru, Kamisu-shi Ibaraki 314-0197 (JP); HOSONO Takahiro, Tainai-shi Niigata 959-2691 (JP)
(74) Representative: D Young & Co LLP
(86) International application number: PCT/JP2015/059455
(87) International publication number: WO 2015/147202

(57) **Abstract**

To provide a polyester polyol which can prevent gelation during the urethane formation reaction in the production of a polyurethane and from which a polyurethane having stable quality can be produced.

A polyester polyol containing 1,3-propanediol having an alicyclic skeleton in a side chain and a dibasic acid component as constituent components, which has an alkali metal content of 20 ppm by mass or less.

## Description

### Technical Field

The present invention relates to a polyester polyol which is used as a starting material of a polyurethane, a production method thereof, a method for producing a polyurethane using a polyester polyol obtained by the method and a polyurethane obtained by the method. More specifically, the polyester polyol of the invention and the production method thereof relate to a technique capable of preventing gelation during urethane formation reaction.

### Background Art

Polyurethanes are used in the fields of paints, adhesives, coatings, elastomers, artificial leather/synthetic leather, foams, resins cured by activation energy rays and the like and are useful resins with a wide range of uses.

In general, polyurethanes are known to have various characteristics depending on the structure of the constituent polyol. A polyester polyol containing an alcohol having an alicyclic skeleton in a side chain as a diol component is highly useful because a polyurethane with excellent vibration control property and excellent hydrolysis resistance can be obtained (PTL 1 and PTL 2). As a method for producing a diol having an alicyclic skeleton in a side chain, a synthesis method from an aldehyde having an alicyclic skeleton and formaldehyde has been proposed (PTL 3).

### Citation List

### Patent Literature

- PTL 1:: JP-A-2002-338650
- PTL 2:: JP-A-11-189637
- PTL 3:: WO01/085657

### Summary of Invention

### Technical Problem

The present inventors have examined the production of a polyurethane using a polyester polyol containing a diol having an alicyclic skeleton in a side chain and a dibasic acid component as constituent components as a polyester polyol having an alicyclic skeleton in a side chain which is useful as a starting material of a polyurethane. As a result, gelation occurred during the reaction, and a polyurethane having stable quality could not be produced. Thus, commercialization was difficult.

Objects of the invention are to solve the problems, to prevent gelation during the urethane formation reaction in the production of a polyurethane and to provide a polyester polyol from which a polyurethane having stable quality can be produced.

### Solution to Problem

As a result of close investigation, the inventors have found that the gelation during the urethane formation reaction is caused by trace amounts of alkali metals which are contained in the polyester polyol and that the alkali metals are derived from 1,3-propanediol having an alicyclic skeleton in a side chain. The inventors have solved the problems using a polyester polyol having a reduced alkali metal content and have completed the invention.

That is, the invention provides [1] to [6] below.
[1] A polyester polyol containing 1,3-propanediol having an alicyclic skeleton in a side chain and a dibasic acid component as constituent components, which has an alkali metal content of 20 ppm by mass or less.
[2] The polyester polyol described in [1], wherein the 1,3-propanediol having an alicyclic skeleton in a side chain is cyclohexane-1,1-dimethanol.
[3] A method for producing a polyurethane by reacting the polyester polyol described in [1] or [2] and a polyisocyanate.
[4] A polyurethane obtained by the production method described in [3].
[5] 1,3-Propanediol having an alicyclic skeleton in a side chain, which has an alkali metal content of 40 ppm by mass or less.
[6] The 1,3-propanediol having an alicyclic skeleton in a side chain described in [5] which is cyclohexane-1,1-dimethanol.

### Advantageous Effects of Invention

When the polyester polyol of the invention is used, the gelation during the urethane formation reaction in the production of a polyurethane can be prevented, and a polyurethane having stable quality can be produced.

### Description of Embodiments

The invention is explained in detail below.

According to the invention, a polyester polyol which contains a dibasic acid component and 1,3-propanediol having an alicyclic skeleton in a side chain as constituent components and which has an alkali metal content of 20 ppm by mass or less is provided. Moreover, a method for producing a polyurethane, a polyurethane and 1,3-propanediol having an alicyclic skeleton in a side chain are provided.

### [Polyester Polyol]

As the dibasic acid component constituting the polyester polyol of the invention, a dibasic acid component which is used for a general polyester polyol can be used without any particular limitation. Examples thereof include: aliphatic dibasic acids such as succinic acid, glutaric acid, adipic acid, pimelic acid, suberic acid, azelaic acid, sebacic acid, dodecanedioic acid, brasylic acid and dimer acid; alicyclic dicarboxylic acids such as 1,4-cyclohexanedicarboxylic acid; aromatic dibasic acids such as phthalic acid, isophthalic acid, terephthalic acid and naphthalenedicarboxylic acid; and the like. Of these examples, adipic acid, azelaic acid, sebacic acid, terephthalic acid, isophthalic acid and naphthalenedicarboxylic acid are preferably used, taking into account the availability and the like. A kind of the dibasic acids may be used alone, or two or more kinds thereof may be used in combination.

The alicyclic skeleton of the 1,3-propanediol having an alicyclic skeleton in a side chain constituting the polyester polyol of the invention is not particularly limited, but an alicyclic skeleton having 3 to 10 carbon atoms is preferable. The number of the alicyclic skeleton(s) in the molecule may be one or may be two or more. Examples of specific compounds include cyclopropane-1,1-dimethanol, cyclobutane-1,1-dimethanol, cyclopentane-1,1-dimethanol, cyclohexane-1,1-dimethanol, 2-methylcyclohexane-1,1-dimethanol, 1-cyclohexene-4,4-dimethanol, cycloheptane-1,1-dimethanol, cyclooctane-1,1-dimethanol, dimethylcyclooctane-1,1-dimethanol and the like. Of these examples, cyclopentane-1,1-dimethanol, cyclohexane-1,1-dimethanol, 2-methylcyclohexane-1,1-dimethanol, 1-cyclohexene-4,4-dimethanol and the like are preferable, taking into account the availability and the like, and cyclohexane-1,1-dimethanol is particularly preferable. A kind of the compounds may be used alone, or two or more kinds thereof may be used in combination.

The 1,3-propanediol having an alicyclic skeleton in a side chain of the invention has an alkali metal content of 40 ppm by mass or less, preferably an alkali metal content of 20 ppm by mass or less, more preferably 8 ppm by mass or less, further preferably 4 ppm by mass or less, particularly preferably 2 ppm by mass or less. The 1,3-propanediol having an alicyclic skeleton in a side chain of the invention is preferably cyclohexane-1,1-dimethanol.

The polyester polyol of the invention can also contain a polyhydric alcohol component (preferably a diol) other than the 1,3-propanediol having an alicyclic skeleton in a side chain as a constituent component. As the other polyhydric alcohol component (preferably a diol), a polyhydric alcohol component (preferably a diol) which is used for a general polyester polyol can also be used. In general, the amount of the other polyhydric alcohol component (preferably a diol) is preferably 50 mol% or less, more preferably 30 mol% or less, based on the 1,3-propanediol having an alicyclic skeleton in a side chain.

Taking into account the case in which the other polyhydric alcohol component (preferably a diol) is contained, the ratio of the amounts of the constituent components of the polyester polyol of the invention, the 1,3-propanediol having an alicyclic skeleton in a side chain and the dibasic acid component, in terms of [(the number of constituent units derived from the 1,3-propanediol having an alicyclic skeleton in a side chain)+(the number of constituent units derived from the other polyhydric alcohol component)]:(the number of constituent units derived from the dibasic acid component), is preferably in the range of 1.4:1 to 1.01:1, more preferably in the range of 1.2:1 to 1.04:1, further preferably 1.1:1 to 1.05:1.

The average molecular weight of the polyester polyol of the invention is not particularly limited but is preferably 300 to 4000, further preferably 350 to 3500, particularly preferably 450 to 3000. When the average molecular weight of the polyester polyol is 300 or more, the hydroxyl group concentration is diluted sufficiently, and gelation does not occur easily during the urethane formation. On the other hand, when the average molecular weight of the polyester polyol is 4000 or less, the viscosity in the dissolved state is low, and handling during the urethane formation becomes easy. In the invention, the average molecular weight means the number average molecular weight calculated from the hydroxyl value of the polyester polyol generated.

The polyester polyol of the invention preferably has a melting point of 25°C or lower. The polyester polyol may be in the form of solid, wax, liquid or the like depending on the structure and the molecular weight, but the form of liquid is superior in view of handling because the time required for dissolution and the energy required for dissolution can be saved.

The alkali metal content of the polyester polyol of the invention is 20 ppm by mass or less, preferably 10 ppm by mass or less, more preferably 4 ppm by mass or less, further preferably 2 ppm by mass or less, particularly preferably 1.5 ppm by mass or less, most preferably 1 ppm by mass or less.

As long as the alkali metal content is controlled at 20 ppm by mass or less, the method for producing the polyester polyol of the invention is not particularly limited. However, by a method for producing a polyester polyol in which a dibasic acid component is used as a starting material and is subjected to transesterification with the 1,3-propanediol having an alicyclic skeleton in a side chain of the invention, a polyester polyol having a low alkali metal content can be produced easily.

The starting materials used for the transesterification between a dialkyl ester of a dibasic acid component and 1,3-propanediol having an alicyclic skeleton in a side chain are explained below.

Examples of the dialkyl ester of a dibasic acid component used in this reaction include: dialkyl esters such as dimethyl esters, diethyl esters, dipropyl esters and dibutyl esters of aliphatic dibasic acids such as succinic acid, glutaric acid, adipic acid, pimelic acid, suberic acid, azelaic acid, sebacic acid, dodecanedioic acid, brasylic acid and dimer acid; dialkyl esters such as dimethyl esters, diethyl esters, dipropyl esters and dibutyl esters of alicyclic dicarboxylic acids such as 1,4-cyclohexanedicarboxylic acid; dialkyl esters such as dimethyl esters, diethyl esters, dipropyl esters and dibutyl esters of aromatic dibasic acids such as phthalic acid, isophthalic acid, terephthalic acid and naphthalenedicarboxylic acid; and the like. Of these examples, dialkyl esters of adipic acid, azelaic acid, sebacic acid, terephthalic acid, isophthalic acid and naphthalenedicarboxylic acid are preferably used, taking into account the availability and the like. Here, the alkali metal content of the dialkyl ester of a dibasic acid component is preferably 2 ppm by mass or less, more preferably 1.5 ppm by mass or less, further preferably 1 ppm by mass or less.

As the 1,3-propanediol having an alicyclic skeleton in a side chain, the 1,3-propanediol having an alicyclic skeleton in a side chain described above can be used.

The polyester polyol of the invention is obtained by esterification or transesterification of the dibasic acid component or the dialkyl ester of a dibasic acid component and the 1,3-propanediol having an alicyclic skeleton in a side chain, which are described above as the starting materials. For the esterification or the transesterification, a method which is generally used for esterification or transesterification in organic synthesis reactions can be applied, and for example, a desired polyester polyol can be obtained by heating and condensing the dibasic acid component and the 1,3-propanediol having an alicyclic skeleton in a side chain. The temperature of the esterification or the transesterification is generally 140 to 240°C, preferably 180 to 220°C. The color hue of the polyester polyol can be maintained excellent by sending an inert gas such as nitrogen or argon into the liquid during the reaction. The esterification or the transesterification may be conducted in the presence of a solvent which does not affect the reaction but in general is preferably conducted without any solvent.

The esterification or the transesterification is preferably conducted in the presence of a catalyst. Preferable catalysts are: titanium compounds such as tetrabutyl titanate, tetraisopropyl titanate, tetra-2-ethylhexyl titanate and titanium acetylacetonate; tin compounds such as dibutyltin oxide, methylphenyltin oxide and hexaethyltin oxide; and magnesium compounds such as magnesium carbonate, magnesium oxide and magnesium alkoxide. Titanium compounds such as tetrabutyl titanate, tetraisopropyl titanate, tetra-2-ethylhexyl titanate and titanium acetylacetonate are more preferable. The amount of the catalyst to be used is not particularly limited but in general is, in terms of the metal atoms based on the polyhydric alcohol(s), preferably in the range of 0.5 to 500 ppm by mass, more preferably in the range of 1 to 100 ppm by mass, particularly preferably 2 to 50 ppm by mass. When the amount of the catalyst is 0.5 ppm by mass or more, the polyester polyol can be generated rapidly, and the amount is economically advantageous because the period of time is shortened. On the other hand, when the amount of the catalyst is 500 ppm by mass or less, the catalyst can be easily removed or deactivated after the reaction.

The catalyst used for producing the polyester polyol also acts as a catalyst in the subsequent urethane formation reaction. Thus, in order to control the reactivity of the subsequent urethane formation reaction, the catalyst is desirably deactivated after the production of the polyester polyol, and the catalyst is desirably deactivated completely. As the method for deactivating the catalyst, a method for deactivating a catalyst used for the production of a general polyester polyol can be applied. For example, when a titanium compound is used as the catalyst, a method for deactivating the catalyst by adding water or a phosphorus compound, a method in which a phosphorus compound is further added after water is added and the like are used. Because the influence of the titanium compound can be reduced sufficiently, a method in which a phosphorus compound is further added after water is added is preferable.

The deactivation method for the case in which a titanium compound is used as the catalyst is explained below.

Heating with the addition of water for deactivating the catalyst is not particularly limited but is in general conducted preferably at a temperature of 70 to 120°C, particularly preferably at a temperature of 90 to 120°C. The heat treatment period is not particularly limited but is in general preferably about one to three hours.

Examples of the phosphorus compound to be added include phosphorous acid, phosphoric acid, dimethyl phosphite, diisopropyl phosphite, di-n-butyl phosphite, isobutyl phosphite, di-n-ethylhexyl phosphite, dilauryl phosphite, dioleyl phosphite, distearyl phosphite, diphenyl phosphite, monomethyl phosphite, monoethyl phosphite, dimethyl phosphate, diethyl phosphate, diisopropyl phosphate, di-n-butyl phosphate, isobutyl phosphate, di-n-ethylhexyl phosphate, dilauryl phosphate, dioleyl phosphate, distearyl phosphate, diphenyl phosphate, monomethyl phosphate, monoethyl phosphate and the like. Of these examples, phosphorous acid, diphenyl phosphite, distearyl phosphite and diphenyl phosphate are preferable. With respect to the amount of the phosphorus compound to be added based on the amount of the titanium compound contained in the polyester polyol, the ratio by mole of titanium atoms in the titanium compound:phosphorus atoms in the phosphorus compound is preferably 1:0.01 to 2.

When the polyester polyol produced in the above manner is used as a starting material of a polyurethane, the polyester polyol produced in the above manner is preferably used after removing water from the polyester polyol. Water is removed preferably after the addition of the phosphorus compound, although the method is not limited thereto, and water may be removed after the heat treatment with the addition of water and before the addition of the phosphorus compound. Water can be removed by any method such as heating and drying at a reduced pressure.

The polyester polyol of the invention can be obtained in this manner.

### [Production Method of Polyurethane]

The method for producing a polyurethane in the invention includes a step of reacting a polyester polyol and a polyisocyanate. The polyurethane of the invention is obtained by the production method.

When a polyurethane is produced using the polyester polyol of the invention, a method which is used for urethane formation reaction of a general polyester polyol can be applied. As the isocyanate, for example, generally used isocyanates such as diphenylmethane-4,4'-diisocyanate (hereinafter abbreviated to MDI), tolylene diisocyanate, 1,5-naphthalene diisocyanate, xylylene diisocyanate, isophorone diisocyanate, hexamethylene diisocyanate and hydrogenated MDI can be used. Also, a chain extender such as a low molecular polyol or polyamine and the like can be used together according to the need. The chain extender is not particularly limited, but a low molecular compound which mainly contains an aliphatic diol having 2 to 20 carbon atoms and which has at least two active hydrogen atoms (hereinafter sometimes simply referred to as "a low molecular compound having active hydrogen atoms") is preferably used. Examples of the low molecular compound having active hydrogen atoms include ethylene glycol, 1,3-propanediol, 1,4-butanediol, 1,5-pentanediol, 1,6-hexanediol, 3-methyl-1,5-pentanediol, 1,8-octanediol, 1,9-nonanediol, xylylene glycol, bis-hydroxy benzene, neopentyl glycol, trimethylolpropane, glycerin, 3,3-dichloro-4,4'-diaminodiphenylmethane, isophorone diamine, 4,4'-diaminodiphenylmethane and the like.

The reactivity of the polyester polyol of the invention can be evaluated for example by reacting the polyester polyol with MDI at 50°C and measuring the apparent reaction rate constant. That the apparent reaction rate constant is large means that the rate of reaction between the polyester polyol and the isocyanate is high. However, because the urethane formation reaction is exothermic reaction, when the reaction rate is too high, the system is locally overheated abnormally, and side reaction occurs at the same time, sometimes causing an undesirable result such as gelation. Accordingly, in order to obtain a polyurethane having stable quality, in general, the apparent reaction rate constant with MDI at 50°C is preferably 0.01 kg·mol⁻¹·min⁻¹ or more and 0.1 kg·mol⁻¹·min⁻¹ or less.

The polyurethane obtained by the method of the invention has excellent mechanical strength and excellent hydrolysis resistance under acidic and basic conditions and can be suitably used for uses such as sheets, films, forms, rolls, gears, solid tires, belts, hoses, tubes, packing materials, vibration proof materials, soles, sports shoes, machine parts, building materials, automotive parts, furniture, linings, sealing materials, waterproof materials, sporting goods, elastic fibers, artificial leather, fiber treating agents, adhesives, coating agents, binders and paints.

### Examples

The invention is explained in further detail below by an Example, but the invention is not limited by the Example. In the Example and the Comparative Example below, the physical property values were measured by the following method.

### [Method for Measuring Reaction Rate Constant]

In a three-necked flask placed in an oil bath at 50°C, 15 mmol of the polyester polyol obtained in the Example or the Comparative Example described below and 15 mmol of MDI (diphenylmethane-4,4'-diisocyanate) were reacted in nitrogen for 30 minutes, and the decrease in the amount of the isocyanate groups in the system was recorded to determine the reaction rate constant (kg.mol⁻¹·min⁻¹). As the reaction rate constant increases, the reaction becomes difficult to control.

### <Example 1>

To a reactor, 133.4 g of adipic acid and 179.4 g of 1,1-cyclohexanedimethanol having a sodium atom content of 7.6 ppm by mass were charged, and the mixture was heated to 200°C in a nitrogen atmosphere at the normal pressure. Esterification was thus conducted while water generated was distilled off from the system. At the point where the distillation of water generated became less, 5 mg of tetraisopropyl titanate was added, and the reaction was continued while reducing the pressure. Next, 5 g of water (corresponding to 2% by weight relative to the theoretical yield) was added, and the mixture was stirred at 100°C for two hours. Then, 0.02 g of diphenyl phosphate was added, and water was distilled off at a reduced pressure. A polyester polyol having a number average molecular weight of 2120 was thus obtained. The sodium atom content of the polyester polyol obtained was 3.7 ppm by mass.

The reaction rate constant of the polyester polyol obtained, which was examined by the above measurement method, was 0.08 kg.mol⁻¹·min⁻¹.

### <Comparative Example 1>

A polyester polyol was obtained in the same manner as in Example 1 except that 1,1-cyclohexanedimethanol having a sodium atom content of 213.6 ppm by mass was used. The sodium atom content of the polyester polyol obtained was 103.2 ppm by mass. When the reaction rate constant was examined by the above measurement method, the internal temperature increased significantly immediately after the initiation of the reaction, and the reaction rate constant could not be measured due to gelation. This Comparative Example shows that the rate of reaction of a polyester polyol obtained using 1,1-cyclohexanedimethanol containing an alkali metal as a starting material with an isocyanate is extremely high and that gelation is caused easily.

Although the invention has been explained in detail and referring to specific embodiments, it is obvious to one skilled in the art that various changes and modifications can be added without departing from the spirit and the scope of the invention.

This application is based on a Japanese patent application filed on March 28, 2014 (patent application No. 2014-70036), and the contents thereof are incorporated herein by reference.

## Claims

1. A polyester polyol containing 1,3-propanediol having an alicyclic skeleton in a side chain and a dibasic acid component as constituent components, which has an alkali metal content of 20 ppm by mass or less.

2. The polyester polyol according to claim 1, wherein the 1,3-propanediol having an alicyclic skeleton in a side chain is cyclohexane-1,1-dimethanol.

3. A method for producing a polyurethane by reacting the polyester polyol according to claim 1 or 2 and a polyisocyanate.

4. A polyurethane obtained by the production method according to claim 3.

5. 1,3-Propanediol having an alicyclic skeleton in a side chain, which has an alkali metal content of 40 ppm by mass or less.

6. The 1,3-propanediol having an alicyclic skeleton in a side chain according to claim 5, which is cyclohexane-1,1-dimethanol.
